# FASCICULE DE BREVET EUROPEEN

(11) **EP 2 636 661 B1**
(45) Date de publication et mention de la délivrance du brevet: **14.07.2021**
(21) Numéro de dépôt: 13290006.9
(22) Date de dépôt: 14.01.2013
(51) Int. Cl.: C07C 1/00, C10G 11/04, C07C 2/12, C10G 11/05

(54) **Procédé de conversion d'une charge lourde, mettant en oeuvre une unité de craquage catalytique et une étape d'hydrogénation sélective de l'essence issue du craquage catalytique**
Umwandlungsverfahren einer schweren Ladung durch Einsatz einer Katkrackeinheit und einer selektiven Hydrierphase des aus dem Katkrackverfahren entstandenen Kraftstoffs
Method for converting a heavy load using a catalytic cracking unit and a step for selective hydrogenation of gasoline from catalytic cracking

(30) Priorité: 15.02.2012 FR 1200424
(43) Date de publication de la demande: 11.09.2013
(73) Titulaire: IFP Energies nouvelles, 92500 Rueil-Malmaison (FR)
(72) Inventeur: Feugnet, Frederic, 69004 Lyon (FR); Hugues, François, 69390 Vernaison (FR); Touchais, Natacha, 38200 Vienne (FR); Dulot, Hugues, 69007 Lyon (FR); Pucci, Annick, 78290 Croissy-sur-Seine (FR)
(74) Mandataire: IFP Energies nouvelles

(56) Documents cités:
- WO-A1-2006/067305
- US-A- 2 945 906
- US-A- 5 177 282
- US-A- 5 202 015
- US-A- 6 143 942
- US-A1- 2011 272 326
- US-B1- 6 803 494
- R. VAN GRIEKEN ET AL.: "NITROGEN AND SULPHUR POISONING IN ALKENE OLIGOMERIZATION OVER NANOSTRUCTURATED ALUMINOSILICATES (Al-MTS, Al-MCM-41) AND NANOCRYSTALLINE n-HZM-5", APPLIED CATALYSIS A: GENERAL, vol. 337, 23 décembre 2007 (2007-12-23), pages 173-183, XP002682828, - DOI: 10.1016/j.apcata.2007.12.011
- E. KRIVÁN ET AL.: "INVESTIGATION OF THE OLIGOMERIZATION OF LIGHT OLEFINS ON ION EXCHANGE RESIN CATALYST", HUNGARIAN JOURNAL OF INDUSTRIAL CHEMISTRY VESZPRÉM, 31 janvier 2010 (2010-01-31), pages 53-57, XP002682829, Extrait de l'Internet: URL:http://konyvtar.uni-pannon.hu/hjic/HJI C38_053_057.pdf
- ALI G. MADHAAH: "A NEW CATALYTIC CRACKING PROCESS TO MAXIMIZE REFINERY PROPYLENE", THE ARABIAN JOURNAL FOR SCIENCE AND ENGINEERING, vol. 33, no. 1B 30 avril 2008 (2008-04-30), pages 17-28, XP002708516, Extrait de l'Internet: URL:http://ajse.kfupm.edu.sa/articles/331B _P.2.pdf
- None

## Description

### DOMAINE DE L'INVENTION

L'invention concerne un procédé de conversion d'une charge hydrocarbonée lourde présentant une grande flexibilité pour la production de distillat moyen, d'essence et de propylène.

Le procédé selon la présente invention fait appel à une unité de craquage catalytique (FCC).

Généralement ces unités de craquage catalytique sont optimisées en vue de la production de produits légers; gaz liquéfiés (ou GPL), oléfines légères, et essence, afin de répondre soit au marché des polymères issus de la polymérisation d'oléfines légères, soit aux besoins de la consommation d'essence du parc automobile.

A l'heure actuelle, étant donnée la forte progression de la diésélisation du parc automobile, la demande en produits de type distillat moyen se trouve largement accrue.

Par conséquent, un autre mode de fonctionnement de l'unité de craquage catalytique a été développé en vue d'orienter la production vers les distillats moyens.

La flexibilité et l'amélioration des rendements vers l'un ou l'autres des trois produits sont obtenues par l'addition d'une unité d'oligomérisation traitant les oléfines C4 à C9 issues du FCC ou d'autres sources additionnelles telles que le l'unité de coking, de visbreaking, de conversion du méthanol en oléfines ou de tout autre procédé de conversion d'alcools en oléfines, de steam-cracking ou en encore de l'unité de synthèse Fischer-Tropsh, ou de deshydrogénation de paraffines, seules ou en mélange.

On peut trouver une description des unités de coking, visbreaking et steam cracking dans l'ouvrage de référence "Raffinage et génie chimique" de P. Wuithier publié aux éditions Technip.

L'unité d'oligomérisation requiert une étape de purification afin de réduire la teneur en composés azotés, qui sont des poisons de la réaction. Les diènes et les composés soufrés présents dans la charge de l'oligomérisation qui sont des inhibiteurs ou des poisons de la réaction, ne sont en général pas réduits dans cette étape de purification, et ont un impact négatif sur la durée de cycle du catalyseur.

La présente invention consiste essentiellement à ajouter une étape d'hydrogénation sélective sur l'essence sortie FCC (donc en amont de l'unité d'oligomérisation) qui permet de limiter le taux de ces inhibiteurs, et de ce fait d'accroître la durée de cycle du catalyseur d'oligomérisation sans altérer la distribution en produits recherchés.

### EXAMEN DE L'ART ANTERIEUR

La demande de brevet FR 2.935.377 concerne un procédé de conversion d'une charge hydrocarbonée dite lourde en vue de coproduire du propylène et de l'essence avec un rendement minimum. Le procédé selon cette invention comprend au moins deux étapes réactionnelles, une première étape de craquage catalytique et une seconde étape d'oligomérisation des oléfines en C3 et C4, ou des oléfines en C4, ou des oléfines en C4 et C5, issues du craquage catalytique.

Le procédé selon le brevet cité permet de réaliser deux types de production correspondant à deux cas de marche distincts:
▪ Une marche dite "maxi propylène" correspondant à une production maximum en propylène tout en maintenant un rendement minimum en essence, voire même légèrement augmenté par rapport au rendement potentiel de l'unité de craquage catalytique seule ou,
▪ Une marche dite "maxi essence" correspondant à une production maximale en essence sans production de propylène.

Dans ce brevet, seules les oléfines en C3, C4 et C5 sont mentionnées.

La demande FR 10/04.585 décrit un procédé de conversion d'une charge lourde permettant d'améliorer la sélectivité en distillat moyen. Le procédé fait appel à une unité de craquage catalytique suivie d'une ou plusieurs unités d'oligomérisation d'oléfines à nombre d'atomes de carbone allant de C2 à C9 permettant de produire préférentiellement une coupe distillat moyen additionnelle. La partie légère de l'oligomérat produit, non incorporable dans la coupe distillat moyen, est recyclée au FCC pour être craquée en oléfines légères qui retournent aux unités d'oligomérisation en supplément des oléfines de la charge afin de former préférentiellement des oligomérats lourds incorporables à la coupe distillat moyen.

Dans cette demande, la coupe C2 à C9 oligomérisée est constituée par une partie des produits du FCC sans aucune mise en œuvre d'autres procédés avant l'oligomérisation.

Le brevet FR 2.797.639B1 décrit un procédé de production d'essence à faible teneur en soufre comprenant une étape d'hydrogénation sélective des dioléfines et éventuellement au moins une étape visant à augmenter le poids moléculaire des produits soufrés légers présents dans l'essence. Le brevet cité décrit une séparation de l'essence en deux fractions : essence légère et essence lourde et permet la production d'essences à faible teneur en soufre.

Le brevet FR 2.895.416B1 décrit un système catalytique permettant de réaliser conjointement l'hydrogénation sélective de composés polyinsaturés en composés mono insaturés contenus dans des essences, ainsi que l'alourdissement des composés soufrés légers par réaction avec les composés insaturés.

Dans les brevets cités, le but recherché est l'obtention d'une essence sans perte notable de l'indice octane et à faible teneur à soufre, l'essence allant au pool carburant.

Dans le cadre de la présente invention, la mise en œuvre d'une unité d'hydrogénation sélective sur la coupe essence issue de l'unité de craquage catalytique avant la séparation entre essence légère et essence lourde, permet d'obtenir une coupe essence légère en C5-C9 à teneur réduite en dioléfines mais aussi en soufre, qui peut être envoyée directement vers l'unité finale de purification (abattement des composés azotés) précédant l'unité d'oligomérisation. Par cette mise en œuvre, la durée de cycle des catalyseurs d'oligomérisation se trouve significativement améliorée sans altérer la distribution en produit recherchés.

### DESCRIPTION SOMMAIRE DES FIGURES

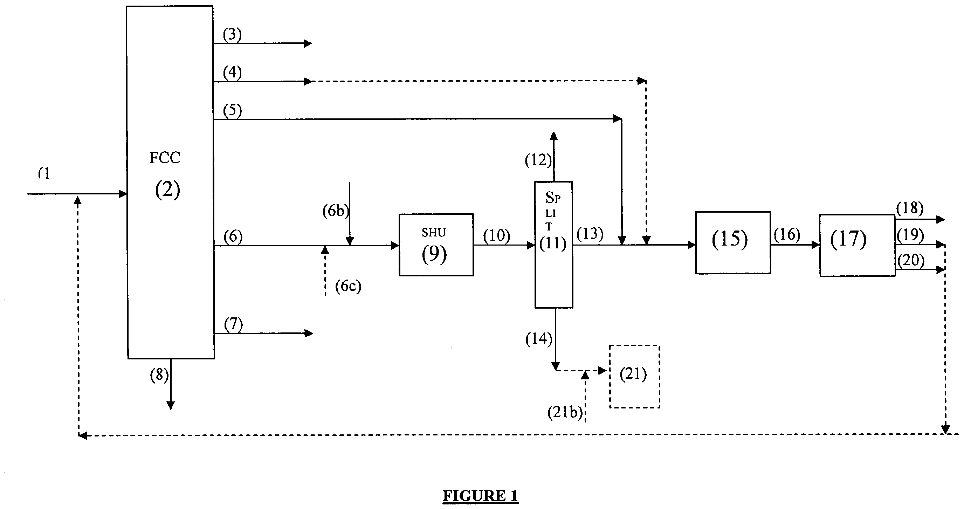

La figure 1 représente un schéma du procédé selon l'invention. Les lignes en pointillés représentent des alimentations ou des recyclages optionnels. Les notations des unités sont introduites dans la suite du texte.

### DESCRIPTION SOMMAIRE DE L'INVENTION

L'invention concerne un procédé de conversion tel que défini à la revendication 1. L'invention consiste en un procédé de conversion d'une charge hydrocarbonée lourde présentant une grande flexibilité pour la production de distillat moyen, d'essence et de propylène, faisant appel à unité de craquage catalytique (FCC), suivie d'une unité d'hydrogénation sélective (SHU) de l'essence issue de l'unité FCC.

L'essence issue de l'unité de craquage catalytique a un point de coupe final différent selon qu'on recherche une marche maxi essence (ne faisant pas partie de l'invention) ou une marche maxi distillat.
- Pour la marche maxi essence (ne faisant pas partie de l'invention), l'essence ex FCC est préférentiellement définie comme C5-220°C
- Pour la marche maxi distillat, l'essence ex FCC est préférentiellement définie comme C5-150°C.

Cette distinction est importante car étant donné la flexibilité du procédé, il convient de bien avoir à l'esprit la définition de l'essence sortie FCC qui est à prendre en compte dans chaque cas.

Le procédé selon l'invention peut également fonctionner selon une marche dite "maxi propylène" qui est compatible avec n'importe quel point de coupe final de l'essence compris entre 150°C et 220°C. Cette marche "maxi propylène" est essentiellement obtenue par les conditions de marche de l'unité de craquage catalytique (conditions dites "haute sévérité"), et l'utilisation d'un catalyseur incorporant une certaine proportion de zéolithe ZSM 5.

L'essence prétraitée (sortie SHU) est alors séparée en deux coupes au travers d'une étape de distillation appelée splitter (SPLIT) :
- une coupe dite essence légère, riche en C5-C6, de nature oléfinique, et à très faible teneur en soufre et en dioléfines, présentant un point de coupe final compris entre 50°C et 80°C,
- une coupe dite essence lourde, ayant pour point initial d'ébullition le point final de la coupe essence légère, et présentant un point final de distillation de 220°C (en marche maxi essence (ne faisant pas partie de l'invention) ou maxi propylène), ou 150°C (en marche maxi distillat), et qui peut être envoyée vers une unité d'hydrodésulfuration pour remplir les spécifications commerciales carburant.

La fraction légère (C5-Pf) de la coupe essence hydrotraitée, c'est à dire principalement la fraction C5-C6, à laquelle on peut éventuellement ajouter une certaine proportion de coupe C4, est alors envoyée vers une section de purification (PUR) en amont de l'unité d'oligomérisation (OLG) afin d'abattre les composés azotés.

En fonction du catalyseur mis en œuvre et des conditions opératoires considérées, l'unité d'oligomérisation (OLG) permet d'orienter la production vers l'essence ou le distillat moyen.
- Dans le mode de fonctionnement maxi propylène, l'ensemble de l'effluent issu de l'étape d'oligomérisation constitué d'une coupe essence légère PI-150°C, essence lourde 150-220°C et d'une coupe (dite "kéro") ayant un point initial d'ébullition supérieur à 220°C (et notée en abrégé 220°C+) est recyclé au FCC.
- Dans le mode de fonctionnement maxi essence (ne faisant pas partie de l'invention), seule la partie "kéro" 220°C + de l'oligomérat est recyclé au FCC, essences légère et lourde étant envoyées au pool essence.
- Dans le mode de fonctionnement maxi distillat, seule la coupe essence légère PI-150°C sera recyclée au FCC. La partie distillat moyen de point initial de distillation supérieur à 150°C, notée 150°C+, nécessite un hydrotraitement final pour être transformée en gazole aux spécifications commerciales.

Le procédé selon la présente invention fait appel à une étape d'hydrogénation sélective (SHU) de la coupe essence issue de l'unité de craquage catalytique (FCC) qui consiste essentiellement à convertir les dioléfines en oléfines et à alourdir les composés soufrés. Ainsi, la coupe essence légère après l'étape de séparation (dite splitter) présente une teneur réduite en composés soufrés et en diènes qui sont les inhibiteurs principaux du catalyseur d'oligomérisation.

L'ajout de l'étape d'hydrogénation sélective (SHU) sur l'essence ex FCC permet ainsi d'augmenter très significativement la durée de cycle de fonctionnement de l'étape d'oligomérisation (OLG) sans altérer la distribution en produit recherchés.

L'unité d'hydrogénation sélective des essences (SHU) permet l'hydrogénation des dioléfines contenues dans l'essence issue de l'unité de craquage catalytique ainsi que la conversion des composés soufrés légers en composés soufrés de point d'ébullition supérieurs. L'hydrogénation étant sélective, les oléfines sont peu hydrogénées.

L'essence ainsi hydrotraitée est alors séparée en deux coupes au niveau du splitter (SPLIT) : une essence légère, à très faible teneur en composés soufrés et riche en oléfines, et une essence lourde qui peut alors envoyée vers une unité d'hydrodésulfuration.

L'essence légère est notée C5- Pf.

L'essence lourde est notée Pf-150°C ou 220°C (respectivement en marche maxi distillat et maxi essence (ne faisant pas partie de l'invention)).

Pf désigne le point de coupe séparant la coupe essence légère et la coupe essence lourde.

Pf est compris entre 50°C et 80°C.

La présente invention est compatible avec toutes les technologies de réacteur de craquage catalytique (FCC), que ce soit une technologie à écoulement gaz solide ascendant, ou à écoulement gaz solide descendant.

L'unité de craquage catalytique (FCC) mis en œuvre dans le présent procédé peut se décliner selon plusieurs modalités: avec un seul réacteur ou plusieurs réacteurs, chaque réacteur pouvant fonctionner en écoulement ascendant ou en écoulement descendant.

Dans le cas de plusieurs unités d'oligomérisation (OLG) associées à l'unité de craquage catalytique (FCC), ces dernières pourront être agencées en série ou en parallèle.

De manière plus précise, la présente invention peut se décrire comme un procédé de conversion d'une charge hydrocarbonée lourde présentant une grande flexibilité pour la production de distillat moyen, d'essence et de propylène faisant appel aux étapes suivantes:
a) une étape de craquage catalytique (FCC) de la coupe lourde produisant une coupe essence C5- 220°C lorsque le FCC est orienté vers la production d'essence, et C5-150°C lorsque le FCC est orienté vers la production de distillat moyen,
b) une étape d'hydrogénation sélective (SHU) de la coupe essence issue de l'unité de craquage catalytique (FCC) fonctionnant aux conditions suivantes: une pression comprise entre 0,5 et 5 MPa, une température comprise entre 80°C et 220°C, avec une vitesse spatiale liquide (LHSV) comprise entre 1h⁻¹ et 10 h⁻¹, la vitesse spatiale liquide étant exprimée en litre de charge par litre de catalyseur et par heure (l/l.h).
c) une étape de séparation par distillation (SPLIT) de l'essence issue de l'étape b) permettant de séparer deux coupes: une coupe essence légère C5-Pf, et une coupe essence lourde Pf-150°C ou Pf-220°C en fonction du point de coupe final de l'essence sortie FCC, la température Pf faisant la démarcation entre essence légère et essence lourde étant comprise entre 50°C et 80°C,
d) une étape de purification (PUR) de l'essence légère C5-Pf issue de l'étape c) qui a pour but d'abattre l'azote à moins de 1ppm poids, de préférence moins de 0,2 ppm,
e) une étape d'oligomérisation (OLG) de l'essence légère C5-Pf issue de l'étape de purification (PUR),
f) une étape de séparation des oligomères obtenus à l'issue de l'étape e) permettant de dégager au moins 2 coupes:
   - une coupe essence C5-150°C
   - une coupe distillat 150°C+

Optionnellement on peut aussi produire un raffinat LPG.

La suite du texte fournit des informations sur les 2 cas de marche du procédé selon l'invention.

### Mode de fonctionnement maxi propylène :

Le mode maxi propylène s'obtient essentiellement par les conditions de marche au FCC dite marche à haute sévérité (température sortie du réacteur supérieur à 600°C) et utilisation d'un catalyseur incorporant une certaine proportion de zéolithe ZSM5.

Dans le mode de fonctionnement maxi propylène, les conditions opératoires de l'étape d'oligomérisation (OLG) sont les suivantes:
- température comprise entre 120°C et 250°C,
- pression comprise entre 3 et 6 MPa,
- catalyseurs à base de silice alumine.

Dans ce mode de fonctionnement, la coupe essence C5-150°C et la coupe distillat 150°C+ issues de l'étape de séparation des oligomères sont recyclées au FCC. Constitués principalement d'oléfines, ces recycles présentent un très fort potentiel en propylène dans les conditions de craquage du FCC.

### Mode de fonctionnement maxi essence (ne faisant pas partie de l'invention):

Dans le mode de fonctionnement maxi essence (ne faisant pas partie de l'invention), les conditions opératoires de l'étape d'oligomérisation (OLG) sont les suivantes:
- température comprise entre 60°C et 350°C, et préférentiellement comprise entre 100°C et 300°C, et de manière encore davantage préférée comprise entre 120°C et 250°C,
- pression comprise entre 1 et 10 MPa (1MPa= 10⁶ pascal), de préférence entre 2 et 8 MPa et de manière encore préférée comprise entre 3 et 6 MPa,
- catalyseurs à base de silice alumine, ou d'alumine silicée amorphe, ou de résine organique acide, ou encore de zéolithes cristallisées. De manière préférée, les catalyseurs sont à base de silice alumine, ou d'alumine silicée amorphe, ou de résine organique acide, et de manière préférée de type résine sulfoniques.

Dans ce mode de fonctionnement, seule la coupe distillat 150°C-360°C issue de l'étape de séparation des oligomères peut être avantageusement recyclée au FCC, afin d'améliorer la production globale d'essence ainsi que la sélectivité essence par rapport au distillat moyen.

### Mode de fonctionnement maxi distillât :

Dans le mode de fonctionnement maxi distillat, les conditions opératoires de l'étape d'oligomérisation (OLG) sont les suivantes:
- température comprise entre 150°C et 350°C,
- pression comprise entre 3 et 6 MPa,
- catalyseur à base de zéolithe cristallisée choisie de préférence parmi les zéolithes suivantes: ferrierite, chabazite, zéolithes Y et US-Y, ZSM-5, ZSM-12, NU-86, mordénite, ZSM-22, NU-10, ZBM-30, ZSM-11, ZSM-57, ZSM-35, IZM-2, ITQ-6 et IM-5, SAPO, prises seules ou en mélange.

De manière très préférée, ladite zéolithe est sélectionnée dans le groupe constitué par les zéolithes ferrierite, ZSM-5, mordénite et ZSM-22, prises seules ou en mélange.

De manière encore plus préférée, la zéolithe utilisée est la ZSM-5.

Dans ce mode de fonctionnement, seule la coupe essence légère PI-150°C peut être avantageusement recyclée au FCC. La partie distillat moyen d'intervalle de distillation compris entre 150°C et 360°C, nécessite un hydrotraitement final pour être transformée en gazole aux spécifications commerciales.

Selon une autre variante de la présente invention, compatible avec toutes les variantes précédemment décrites, les étapes b) d'hydrogénation sélective et c) de séparation en essence légère et essence lourde sont conduite dans une seule unité de distillation réactive.

Dans le cas où la teneur en azote de l'essence légère issue de l'étape de séparation (SPLIT) est inférieure à 1 ppm, et préférentiellement inférieure à 0,2 ppm, l'étape de purification (PUR) située en amont de l'étape d'oligomérisation (OLG) peut être supprimée.

### DESCRIPTION DETAILLEE DE L'INVENTION

L'invention concerne un procédé de conversion d'une charge hydrocarbonée dite lourde, c'est à dire constituée d'hydrocarbures de température d'ébullition supérieure à environ 340°C.

La coupe hydrocarbonée lourde de départ peut être un résidu atmosphérique ou un distillat sous vide.

Le procédé selon l'invention comprend une étape de craquage catalytique (FCC), une unité d'hydrogénation sélective (SHU) de l'essence produite à partir de l'unité de craquage catalytique, une étape de séparation de l'essence produite (SPLIT) en une coupe essence légère, à faible teneur en composés soufrés et dioléfines, et une coupe essence lourde, et au moins une étape d'oligomérisation (OLG) de l'essence légère.

De façon optionnelle, l'oligomérisation de la coupe essence légère peut également se faire en mélange avec tout ou partie des coupes C3 et C4 issues du FCC ou d'autres sources additionnelles telles que le l'unité de coking, de "visbreaking", de conversion du méthanol en oléfines ou de tout autre procédé de conversion d'alcools en oléfines, de stem-cracking ou en encore de l'unité de synthèse Fischer-Tropsh ou de déshydrogénation de paraffines , seules ou en mélange.

L'hydrogénation sélective (SHU) permet d'hydrogéner d'une part les dioléfines en oléfines, en limitant de façon notable l'hydrogénation des oléfines en paraffines.

De plus, elle permet de convertir les composés soufrés légers en composés soufrés plus lourds.

La séparation entre la coupe essence légère commençant en C5, et l'essence lourde de point final d'ébullition 150°C ou 220°C (selon respectivement la marche maxi distillat, ou maxi essence (ne faisant pas partie de l'invention)) est réalisée dans une distillation (notée SPLIT sur la figure 1).

Selon la présente invention, le point de coupe Pf séparant la coupe essence légère de la coupe essence lourde est compris entre 50°C et 80°C.

La coupe essence légère, d'intervalle de distillation C5-Pf qui est envoyée à l'oligomérisation (OLG) contient :
- moins de 10 atomes de carbones et de façon préférée moins de 7 atomes de carbones.
- moins de 100 ppm (poids) de soufre et de façon préférée moins de 50 ppm, et de manière encore plus préférée moins de 10 ppm (poids) .
- moins de 2000 ppm (poids) de dioléfines, et de façon préférée moins de 1000 ppm, et de manière encore plus préférée moins de 500 ppm (poids).

Cette coupe légère riche en oléfines peut alors être envoyée vers l'unité d'oligomérisation (OLG) afin de transformer les oléfines contenues en oléfines plus lourdes, pouvant être utilisées en tant qu'essence ou distillats moyens, et ce en fonction du catalyseur et des conditions opératoires mises en œuvre sur cette étape.

La séparation au niveau du splitter (SPLIT) entre essence légère et essence lourde permet :
- de valoriser les oléfines contenues dans l'essence issue du craquage catalytique, qui sont majoritairement des oléfines en C5-Pf. Cette séparation permet de concentrer les oléfines réactives vis à vis de l'oligomérisation ultérieure, dans l'essence légère, et donc d'envoyer celle ci directement sur l'unité d'oligomérisation (OLG).

La capacité de l'unité d'oligomérisation (OLG) peut donc être réduite, ce qui est bénéfique en terme d'investissement et de coûts opératoires. En effet, la réactivité des oléfines en oligomérisation décroît fortement avec le nombre d'atomes de carbone.
- de concentrer les composés soufrés dans la coupe essence lourde, la SHU ayant alourdi les composés soufrés qui se retrouvent alors dans ladite essence lourde typiquement envoyée à un hydrotraitement sélectif.

La suite de la description donne des informations sur les différentes unités constituant l'enchainement d'unités selon la présente invention.

### Unité FCC :

Généralement la charge de l'unité de craquage catalytique est constituée d'un distillat sous vide, ou éventuellement d'un résidu atmosphérique. La charge globale craquée contient 100 % poids d'hydrocarbures ayant un point d'ébullition supérieur à 340°C.

Dans certains cas correspondant à des charges de l'unité FCC très chargées en métaux, composés soufrés et azotés, un prétraitement peut être mis en place en amont de l'unité FCC.

Ce prétraitement amont FCC (non représenté sur la figure 1) permet d'une part d'améliorer l'activité de la SHU en amont de l'unité d'oligomérisation (OLG), d'autre part de déplacer le point de coupe entre essence légère et essence lourde au niveau du splitter (SPLIT).

Par ce déplacement du point de coupe aux environs de 150°C, il est ainsi possible de maximiser la charge allant à l'unité d'oligomérisation (OLG).

Les essences issues d'unités de craquage sont généralement riches en mono-oléfines (entre 20 % et 50% poids) et en soufre (entre 100 ppm et 2 % poids de soufre), mais également en dioléfines dont la teneur peut varier de 1% poids à 5% poids.

Le fractionnement des effluents du craquage catalytique (FCC) permet d'obtenir plusieurs coupes :
- une coupe C3 riche en composés contenant 3 atomes de carbone et en particulier le propylène,
- une coupe C4 riche en composés contenant 4 atomes de carbone et en particulier les butènes,
- une coupe essence contenant des oléfines dont le nombre de carbone est égale ou supérieur à 5 atomes, et dont le point d'ébullition final est de 220°C lorsqu'on cherche une marche maxi essence (ne faisant pas partie de l'invention), et 150°C lorsqu'on cherche une marche maxi distillat.

On note en abrégé cette coupe essence C5-220°C (marche maxi essence (ne faisant pas partie de l'invention)) ou C5 -150°C (marche maxi distillat).

L'unité de craquage catalytique (FCC) comporte un réacteur qui peut être à écoulement ascendant ou à écoulement descendant.

Lorsque le craquage catalytique est effectué dans un seul réacteur à écoulement ascendant, la température de sortie réacteur (ROT) est comprise entre 450°C et 650°C, préférentiellement comprise entre 470°C et 620°C, et le rapport C/O est compris entre 2 et 20, et préférentiellement compris entre 4 et 15.

Lorsque le réacteur est à écoulement descendant, la température de sortie du réacteur (ROT) est comprise entre 480°C et 650°C, et le rapport C/O est compris entre 10 et 50.

Selon l'invention, le catalyseur de craquage catalytique est constitué d'une matrice d'alumine, de silice ou de silice alumine avec ou sans une zéolithe de type Y ultra stable dispersée dans cette même matrice.

L'ajout d'un additif à base de zéolithe ZSM5, la quantité en cristaux de ZSM5 dans l'inventaire total de l'unité de craquage catalytique (FCC) étant inférieure à 30% poids, peut également être envisagé.

Le catalyseur du réacteur FCC est typiquement constitué de particules de diamètre moyen généralement compris entre 40 et 140 micromètres, et le plus souvent compris entre 50 et 120 micromètres.

Le catalyseur peut comprendre en outre au moins une zéolithe présentant une sélectivité de forme de l'un des types structuraux suivants : MEL (par exemple ZSM-11), MFI (par exemple ZSM-5), NES, EUO, FER, CHA (par exemple SAPO-34), MFS, MWW.

Il peut également comprendre l'une des zéolithes suivantes: NU-85, NU-86, NU-88 et IM-5, qui présentent également une sélectivité de forme.

L'avantage de ces zéolithes présentant une sélectivité de forme est l'obtention d'une meilleure sélectivité propylène / isobutène, c'est à dire un rapport propylène / isobutène plus élevé dans les effluents de l'unité de craquage catalytique (FCC).

La proportion de zéolithe présentant une sélectivité de forme par rapport à la quantité totale de zéolithe peut varier en fonction des charges utilisées et de la structure des produits recherchés. Souvent, on utilise de 0,1% à 60 %, préférentiellement de 0,1% à 40 %, et en particulier de 0,1% à 30 % poids de zéolithe présentant une sélectivité de forme.

La ou les zéolithes peuvent être dispersées dans une matrice à base de silice, d'alumine ou de silice alumine, la proportion de zéolithe (toutes zéolithes confondues) par rapport au poids du catalyseur étant souvent comprise entre 0,7% et 80% poids, de préférence entre 1% et 50% poids, et de manière encore préférée entre 5% et 40 % poids.

Dans le cas où plusieurs zéolithes sont utilisées, elles peuvent être incorporées dans une seule matrice ou dans plusieurs matrices différentes. La teneur en zéolithe présentant une sélectivité de forme dans l'inventaire totale est inférieure à 30% poids.

Le catalyseur utilisé dans le réacteur de craquage catalytique (FCC) peut être constitué d'une zéolithe de type Y ultra stable dispersée dans une matrice d'alumine, de silice, ou de silice alumine, à laquelle on ajoute un additif à base de zéolithe ZSM5, la quantité en cristaux de ZSM5 dans l'inventaire total étant inférieure à 30% poids.

### Unité d'hydrogénation sélective (SHU)

La coupe essence, riche en oléfines mais contenant des dioléfines et des composés soufrés est alors envoyée vers une unité d'hydrogénation sélective (SHU). Sur cette unité, les dioléfines sont hydrogénées en présence d'hydrogène en oléfines sans hydrogénation importante des oléfines, et les composés soufrés légers sont convertis en composés soufrés de point d'ébullition supérieur.

Le brevet FR2.935.389 A1 décrit un système catalytique mis en œuvre dans le procédé d'hydrogénation sélective de l'essence.

Le catalyseur mis en forme sur l'étape d'hydrogénation sélective peut également être celui décrit dans le brevet FR2.935.389B1. Il contient au moins un métal du groupe VIb et au moins un métal non noble du groupe VIII déposés sur un support poreux, et dans lequel :
- la teneur en poids d'oxyde de l'élément du groupe VIb est comprise entre 4 et 20% poids,
- la teneur en poids d'oxyde de l'élément du groupe VIII est inférieure à 15% poids,

L'hydrogénation sélective (SHU) consiste à faire passer sur l'un des catalyseurs définis ci-dessus un mélange constitué de l'essence à traiter et d'hydrogène.

L'hydrogène est introduit généralement en faible excès, jusqu'à 5 mole par mole, par rapport à la stœchiométrie, nécessaire pour hydrogéner les dioléfines (une mole d'hydrogène par mole de dioléfine).

Le mélange constitué de l'essence et de l'hydrogène est mis en contact avec le catalyseur sous une pression comprise entre 0,5 et 5 MPa, une température comprise entre 80°C et 220°C, avec une vitesse spatiale liquide (VVH) comprise entre 1h⁻¹ et 10 h⁻¹, la vitesse spatiale liquide étant exprimée en litre de charge par litre de catalyseur et par heure (l/l.h).

### Unité de séparation (SPLIT)

Cette coupe essence sélectivement hydrogénée est alors envoyée vers une colonne de séparation (notée SPLIT) afin de séparer l'essence en deux coupes : une coupe dite essence légère et une coupe dite essence lourde.

La coupe essence légère, C5-Pf contient
- moins de 10 atomes de carbone, et de façon préférée moins de 7 atomes de carbone.
- moins de 100 ppm (poids) de soufre et de façon préférée moins de 50 ppm et encore plus préférée moins de 10 ppm(poids).
- moins de 2000 ppm (poids) de dioléfines, et de façon préférée moins de 1000 ppm, et de manière encore davantage préférée moins de 500 ppm (poids). La coupe essence légère est envoyée à l'unité d'oligomérisation (OLG).

La coupe essence lourde est une coupe Pf- 220°C dans laquelle les composés soufrés ont été concentrés. Cette coupe essence lourde est alors envoyée vers une unité d'hydrodésulfuration, avant d'être envoyée vers le pool essence.

Les coupes C3 et C4 produites à partir de l'unité de craquage catalytique (FCC) peuvent également être envoyées, en partie ou en totalité, vers l'unité d'oligomérisation (OLG), selon les besoins de la raffinerie.

De façon préférée, le propylène contenu dans la coupe C3 est valorisé en tant que produit, tandis que la coupe C4 est envoyée, en mélange avec l'essence légère en C5-C6 vers le prétraitement de l'unité d'oligomérisation (OLG).

La séparation entre l'essence légère et l'essence lourde après l'étape d'hydrogénation sélective est réalisée de préférence au moyen d'une colonne de distillation classique appelée aussi "splitter".

Cette colonne opère généralement à une pression comprise entre 0,1 et 2 MPa, et de préférence entre 0,2 et 1 MPa. Le nombre de plateaux théoriques de cette colonne de séparation est généralement compris entre 10 et 100, et de préférence entre 20 et 60.

Le taux de reflux, exprimé comme étant le reflux externe en kg/h divisé par le débit d'alimentation de la colonne exprimé en kg/h, est généralement inférieur à l'unité et de préférence inférieur à 0,7.

La teneur en soufre de l'essence légère issue de l'unité de séparation (SPLIT) qui est ensuite envoyée vers l'oligomérisation (OLG) est inférieure à 100 ppm, préférentiellement inférieure à 50 ppm, et de manière encore préférée inférieure à 10 ppm.

### Unité de purification (PUR)

L'enchaînement hydrogénation sélective (SHU) et séparation d'une coupe essence légère (SPLIT), permet de préparer une charge d'oligomérisation présentant une teneur réduite en dioléfines et en composés soufrés, inhibiteurs. Elle ne permet généralement pas de réduire la teneur en composés azotés, qui sont des poisons des catalyseurs d'oligomérisation. Il est alors nécessaire d'insérer une étape de purification spécifique pour réduire le taux d'azote total à moins de 1 ppm poids.

Cette étape de purification (PUR) fait appel à toute technique connue de l'homme de l'art, et en particulier peuvent être mis en œuvre :
- des adsorbants tels les alumines, les silices alumines ou les tamis moléculaires, ces derniers préférentiellement à base de zéolithes de type NaX ou NaY.
- un lavage à l'eau préalable, suivi d'une adsorption sur les adsorbants cités plus haut, et préférentiellement sur tamis moléculaires

### Unité d'oligomérisation (OLG)

L'oligomérisation se distingue de la polymérisation par une addition de molécules en nombre limité. Le nombre de molécules s'additionnant est, dans le contexte de l'invention, compris entre 2 et 10, bornes comprises, et généralement entre 2 et 5.

Les oligomérats peuvent cependant comprendre des traces d'oléfines ayant été oligomérisées avec un nombre de molécules supérieur à 10. Le plus souvent, ces traces représentent moins de 5 % poids par rapport aux oligomères formés.

L'oligomérisation peut être réalisée en une ou plusieurs étapes, avec un ou plusieurs réacteurs agencés en parallèle ou en série, et un ou plusieurs catalyseurs. La description suivante du catalyseur et des conditions opératoires peut s'appliquer à l'une quelconque des étapes et/ou à l'un quelconque des réacteurs.

En mode de fonctionnement maxi essence (ne faisant pas partie de l'invention) pour l'oligomérisation, les catalyseurs préférés sont les_catalyseurs à base de silice alumine ou d'alumine silicée amorphe, ou de résine organique acide, de manière préférée de type résine sulfoniques, et les conditions opératoires sont les suivantes:
- température comprise entre 60°C et 300°C, et préférentiellement comprise entre 80°C et 250°C,
- pression comprise entre 1 et 10 MPa (1MPa= 10⁶ pascal), de préférence entre 2 et 8 MPa et de manière encore préférée comprise entre 3 et 6 MPa,

En mode de fonctionnement maxi distillat pour l'oligomérisation, les catalyseurs sont les catalyseurs à base de zéolithes cristallisées choisies de préférence parmi les zéolithes suivantes : ferrierite, chabazite, zéolithes Y et US-Y, ZSM-5, ZSM-12, NU-86, mordénite, ZSM-22, NU-10, ZBM-30, ZSM-11, ZSM-57, ZSM-35, IZM-2, ITQ-6 et IM-5, SAPO, prises seules ou en mélange.

De manière très préférée, ladite zéolithe est sélectionnée dans le groupe constitué par les zéolithes ferrierite, ZSM-5, mordénite et ZSM-22, prises seules ou en mélange.

De manière encore plus préférée, la zéolithe utilisée est la ZSM-5.

Les conditions opératoires de l'oligomérisation (OLG) dans le mode de fonctionnement maxi distillat sont les suivantes :
- température comprise entre 150°C et 350°C,
- pression comprise 3 et 6 MPa.

Les oligomérats formés à l'issue de l'unité d'oligomérisation (OLG) sont alors séparés par distillation en au moins 2 coupes, une coupe essence, une coupe distillats moyens.

La coupe essence présente un point d'ébullition inférieur à 220°C, et préférentiellement inférieur à 150°C.

La coupe distillat moyen présente un point d'ébullition initial supérieur à 130°C et préférentiellement supérieur à 150°C.

De façon optionnelle, une partie de l'essence produite à l'issue de l'étape d'oligomérisation peut être recyclée au réacteur d'oligomérisation de façon à augmenter la production en distillats moyens.

Les oléfines sont oligomérisées pour obtenir un mélange d'hydrocarbures contenant des mono-oléfines avec un nombre d'atomes de carbone majoritairement supérieur ou égal à 8.

Typiquement, à partir d'oléfines C4, on obtient des oligomères dont le nombre d'atomes de carbone est en grande partie inférieur ou égal à 30, et pour la plus grande partie compris entre 8 et 20.

La figure 1 représente le schéma du procédé selon la présente invention.

La charge (1) est introduite dans l'unité de craquage catalytique FCC (2) de laquelle on extrait par ordre de poids moléculaire croissant:
- une coupe gaz sec (3) constituée d'hydrogène (H2), de méthane et éventuellement d'éthane, d'éthylène et propane,
- une coupe C3 (4) formée de molécules d'hydrocarbures contenant 3 atomes de carbone, riche en propylène,
- une coupe C4 (5) formée de molécules d'hydrocarbures contenant 4 atomes de carbone, riche en butènes,
- une coupe essence (6) d'intervalle de distillation compris entre 25°C et 220°C,
- une coupe dite "distillats moyens" (7), d'intervalle de distillation compris entre 220°C et 360°C,
- une coupe (8) dite "slurry" qui rejoint le pool fuel.

La coupe essence (6), en mélange avec un appoint d'hydrogène (6b) et /ou une source secondaire d'oléfines (6c) est envoyée vers l'unité d'hydrogénation sélective d'essence (9) dont l'effluent (10) est séparé dans une colonne à distiller (11) en plusieurs coupes :
- une purge (12) composée de légers (Hydrogène, gaz de craquage tels que méthane, éthane
- une coupe essence légère (13) composée d'hydrocarbures en C5 jusqu'à un point final d'ébullition de Pf °C,
- une coupe essence lourde (14) composés d'hydrocarbures d'intervalle de distillation compris entre Pf °C et 220°C.

La valeur de la température Pf, faisant point de coupe entre essence légère et essence lourde est comprise entre 50°C et 80°C.

La coupe essence légère (13) est alors envoyée en mélange avec la coupe C4 (5) vers un ensemble d'unités de purification (15) qui aboutissent à la charge (16) de l'unité d'oligomérisation (17).

Optionnellement, la coupe C3 (4) peut être mélangée avec la coupe essence légère pour être oligomérisée.

De cette unité d'oligomérisation (17) on extrait au moins 2 coupes:
- une coupe (18) dite raffinat qui correspond aux oléfines non converties et aux paraffines des charges C3 (4) et C4 (5).
- une coupe essence (19) qui correspond aux paraffines contenues dans la coupe (13) ainsi qu'à une partie des oligomérats formés provenant des coupes (5) et (4) et (13).
- une coupe distillats moyens (20) correspondant aux oligomérats lourds formés à partir des coupes (4), (5), (13) d'intervalle de distillation 150°C-360°C qui est envoyé vers un hydrotraitement en vue de produire un gazole aux spécifications commerciales.

La coupe essence lourde (14) peut être envoyée de façon optionnelle en appoint avec de l'hydrogène (21b) vers une unité d'hydrodésulfuration de l'essence (21).

### EXEMPLES

Les exemples qui suivent ont été conçus pour illustrer les différentes cas de marche du procédé selon la présente invention, plus particulièrement au niveau de l'unité d'oligomérisation (OLG), et pour mettre en évidence l'intérêt de la mise en œuvre de l'hydrogénation sélective (SHU) en amont de ladite unité d'oligomérisation.

Les exemples sont associés de la façon suivante:
Exemple A (avec SHU) /Exemple B (sans SHU).
Exemple C (avec SHU)/Exemple D (sans SHU).
Exemple E (avec SHU).

### Exemple A (ne faisant pas partie de l'invention)

Dans cet exemple l'unité d'oligomérisation (OLG) de l'essence légère fait appel à un catalyseur de type résine organique acide (résine sulfonique).

Le schéma est le suivant : hydrogénation sélective (SHU), splitter (SPLIT), oligomerisation (OLG) sur les C5/C6 récupérés en tête du splitter et fractionnement pour séparer l'essence résiduelle 150-°C de la fraction lourde 150°C+ envoyée à un hydrotraitement de Kérosène conventionnel existant.

L'hydrogénation sélective (SHU) est conduite à 160°C, sous une pression de 2,0 MPa, à une vitesse spatiale de 3h-1.

Le rapport H2/Charge, exprimé en litre d'hydrogène par litre de charge est de 5 l/l et le catalyseur est le catalyseur HR 845 commercialisé par la société Axens.

L'unité d'oligomérisation (OLG) de l'essence légère fait appel à un catalyseur de type résine organique acide (résine sulfonique).

Il s'agit du catalyseur commercialisé par Axens sous le nom TA 801. Il est mis en œuvre dans les conditions suivantes : réacteur en lit fixe contenant 50 tonnes de catalyseur, pression fixée à 3 MPa, température croissante entre 60°C et 160°C afin de maintenir une production constante d'oligomères entre début et fin de cycle. La durée du cycle est de 2 mois.

Les propriétés des charge et produits sont données dans le tableau 1 ci dessous:

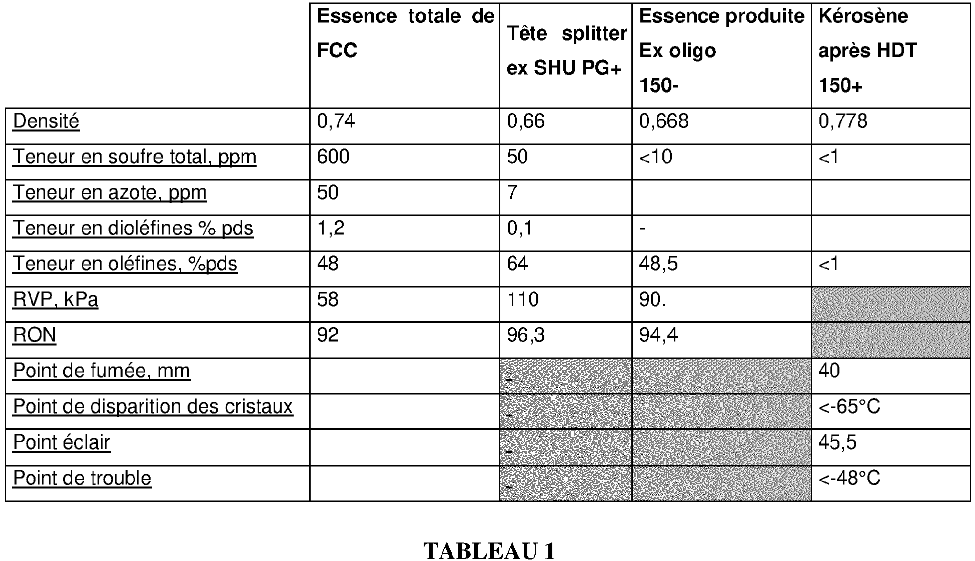

Le bilan matière basé sur l'essence légère décrite ci-dessus est donné dans le tableau 2 ci dessous. Cet exemple montre clairement l'intérêt de l'hydrogénation sélective (SHU) sur les performances.

**TABLEAU 2**

| ***Charge t*/*h*** | | |
|---|---|---|
| | Essence de FCC totale | 80 |
| | Essence légère vers Oligomérisation | 25 |

| ***Produits, t*/*h*** | | |
|---|---|---|
| | Essence produite 150-°C | 17 |
| | Kérosène produit | 8 |

### Exemple B (selon l'art antérieur)

Cet exemple selon l'art antérieur est destiné à mettre en évidence l'intérêt de l'introduction de l'unité d'hydrogénation sélective (SHU) sur l'essence ex FCC. Il est à rapprocher de l'exemple A ci dessus.

Le schéma est le suivant : splitter (SPLIT), oligomerisation (OLG) sur les C5/C6 récupérés en tête du splitter et fractionnement pour séparer l'essence résiduelle 150-°C de la fraction lourde 150°C+ envoyée à un hydrotraitement de Kérosène conventionnel existant

Par rapport à l'exemple A, l'hydrogénation sélective n'existe pas.

Les propriétés des charges sont données dans le tableau 3 ci dessous.

On remarque que la charge de l'unité d'oligomérisation (OLG) est bien plus riche en soufre et en diènes que dans le cas A.

**TABLEAU 3**

| | **Essence totale de FCC** | **Tête Splitter Sans SHU** |
|---|---|---|
| Densité | 0,74 | 0,66 |
| Teneur en soufre total. ppm | 600 | 210 |
| Teneur en azote. ppm | 50 | 7 |
| Teneur en dioléfines % pds | 1,2 | 1,0 |
| Teneur en oléfines. %pds | 48 | 64 |
| RVP. kPa | 58 | 110 |
| RON | 92 | 96,3 |
| Point de fumée, mm | | |
| Point de disparition des cristaux | | |
| Point éclair | | |
| Point de trouble | | |

Le catalyseur d'oligomérisation TA 801 est mis en œuvre dans les mêmes conditions que dans l'exemple A. La température de début de cycle est cette fois de 100°C, et la durée de cycle de moins de 1 mois.

La distribution et la qualité des produits sont les mêmes que dans l'exemple A selon l'invention.

### EXEMPLE C (selon l'invention)

Dans cet exemple, l'unité d'oligomérisation (OLG) de l'essence légère fait appel à un catalyseur à base d'alumine-silicée amorphe.

Le schéma est le suivant: hydrogénation sélective(SHU)/ splitter(SPLIT) et élimination des composés azotés sur tamis moléculaire 13X sur les C5/C6 récupérés en tête de splitter puis oligomerisation (OLG) sur Silice alumine et fractionnement pour séparer l'essence résiduelle 150-°C de la fraction lourde 150+ envoyée à un hydrotraitement existant.

L'hydrogénation sélective est conduite dans les mêmes conditions que dans l'exemple A.

Le splitter est réglé pour obtenir la coupe de tête indiquée dans le tableau ci-dessous, ayant une teneur en impuretés réduite. Les composés azotés sont captés par la mise en œuvre d'un lit fixe opérant à 30°C et contenant un tamis moléculaire (zéolithe NaX), jusqu'à atteindre une teneur résiduelle en azote inférieure à 1 ppm.

L'unité d'oligomérisation de l'essence légère fait appel à un catalyseur constitué de Silice - Alumine Il s'agit du catalyseur commercialisé par Axens sous le nom IP 811.

Il est mis en œuvre dans les conditions suivantes : réacteur en lit fixe contenant 50 tonnes de catalyseur, pression fixée à 6 MPa, température croissante entre 120 et 250°C entre début et fin de cycle, afin de maintenir une production constante d'oligomères.

La durée du cycle est de 3 mois.

Les propriétés des charges et produits sont données dans le tableau 4 ci dessous.

**TABLEAU 4**

| | **Essence totale de FCC** | **Tête splitter ex SHU PG+** | **Essence produite Ex oligo** | **Kérosène après HDT** |
|---|---|---|---|---|
| Densité | 0,74 | 0,655 | 0,66 | 0,783 |
| Teneur en soufre total, ppm S | 600 | 15 | 10 | <1 |
| Teneur en azote, ppm N | 50 | 5 | | |
| Teneur en dioléfines % pds | 1,2 | 0,05 | | |
| Teneur en oléfines. %pds | 48 | 65 | 29 | <1 |
| RVP. kPa | 58 | | | |
| RON | 92 | | | |
| Point de fumée, mm | | | | |
| Point de disparition des cristaux | | | | |
| Point éclair | | | | |
| Point de trouble | | | | |

Le bilan matière basé sur l'essence légère est donné dans le tableau 5 ci dessous.

Cet exemple montre également clairement l'intérêt de l'hydrogénation sélective (SHU) sur les performances.

**TABLEAU 5**

| ***Charge t*/*h*** | | |
|---|---|---|
| | Essencede FCC totale | 86 |
| | Essence légère | 25 |

| ***Produits, t*/*h*** | | |
|---|---|---|
| | Essence produite 150- °C | 14 |
| | Kérosène produit | 11 |

### Exemple D (selon l'art antérieur)

Dans ce cas, le schéma est le suivant : splitter (SPLIT) et oligomerisation sur les C5/C6 récupérés en tête du splitter et fractionnement pour séparer l'essence résiduelle 150-°C de la fraction lourde 150°C+ envoyée à un hydrotraitement existant.

Par rapport à l'exemple C, l'hydrogénation sélective n'existe pas.

Les propriétés des charges sont données dans le tableau 6 ci dessous.

On remarque que la charge de l'unité d'oligomérisation est bien plus riche en soufre et en diènes que dans le cas C.

Le catalyseur d'oligomérisation IP 811 est mis en œuvre dans les mêmes conditions que dans l'exemple C.

La température de début de cycle est cette fois de 210°C, et la durée de cycle de 20 jours.

La distribution et la qualité des produits sont les mêmes que dans l'exemple C selon l'invention.

**TABLEAU 6**

| | **Essence totale deFCC** | **Tête Splitter Sans SHU** |
|---|---|---|
| Densité | 0,74 | 0,655 |
| Teneur en soufre total. ppm | 1200 | 180 |
| Teneur en azote, ppm | 50 | 5 |
| Teneur en dioléfines. %wt | 1,5 | 1,0 |
| Teneur en oléfines. %wt | 48 | 65 |
| RVP, kPa | 58 | 115 |
| RON | 92 | 96,5 |

### Exemple E (selon l'invention)

Dans cet exemple, l'unité d'oligomérisation (OLG) de l'essence légère fait appel à un catalyseur à base de zéolithe.

Le schéma est le suivant: hydrogénation sélective (SHU), splitter (SPLIT), et élimination des azotés sur tamis moléculaire 13X sur les C5/C6 récupérés en tête de splitter, puis oligomerisation (OLG) sur zéolithe et fractionnement pour séparer l'essence résiduelle 150-°C de la fraction lourde 150+ envoyée à un hydrotraitement DHT existant pour produire un diesel de haute qualité.

L'hydrogénation sélective (SHU) est conduite dans les mêmes conditions que dans l'exemple A.

Le splitter est réglé pour obtenir la coupe de tête indiquée dans le tableau ci-dessous, ayant une teneur en impuretés réduite, comme dans l'exemple C.

Les composés azotés sont captés par la mise en œuvre d'un lit fixe contenant du tamis moléculaire (zéolithe Na X), jusqu'à une teneur résiduelle en azote inférieure à 1 ppm. L'unité d'oligomérisation de l'essence légère fait appel à un catalyseur à base de zéolithe commerciale ZSM-5.

Il est mis en œuvre dans les conditions suivantes : réacteur en lit fixe contenant 40 tonnes de catalyseur, pression fixée à 6 MPa, température croissante entre 200°C et 330°C entre début et fin de cycle, afin de maintenir une production constante d'oligomères.

La durée du cycle est de 60 jours.

Les propriétés des charges et produits sont données dans le tableau 7 ci dessous.

**TABLEAU 7**

| | **Essence totale de FCC** | **Tête splitter ex SHU PG+** | **Essence produite 150-** | **Diesel après HDT 150 +** |
|---|---|---|---|---|
| Densité | 0,74 | 0,655 | 0,695 | 0,797 |
| Teneur en soufre total, ppm | 1200 | 15 | 10 | <1 |
| Teneur en azote, ppm | 50 | 5 | | <1 |
| Teneur en dioléfines. %wt | 1.5 | 0.05 | | |
| Teneur en oléfines, %wt | 48 | 65 | 26 | <1 |
| RVP, kPa | 58 | 115 | | |
| RON | 92 | 96,5 | | |
| Point de disparition des cristaux | | | | <-50°C |
| Point éclair | | | | 65 |
| Point de trouble | | | | <-48°C |
| Cétane moteur | | | | 50 |

Le bilan matière est donné dans le tableau 8 ci dessous.

**TABLEAU 8**

| ***Charge t*/*h*** | | |
|---|---|---|
| | Essence de FCC totale | 86 |
| | Essence légère | 25 |

| ***Produits, t*/*h*** | | |
|---|---|---|
| | Essence produite 160-°C | 13 |
| | Diesel produit | 12 |

## Revendications

1. Procédé de conversion d'une charge hydrocarbonée lourde constituée de composés ayant des points d'ébullition supérieur à 340°C, ledit procédé présentant une grande flexibilité pour la production de propylène, d'essence et de distillat moyen faisant appel aux étapes suivantes lorsque le procédé fonctionne en marche maxi propylène ou maxi distillât :
a) une étape de craquage catalytique (FCC) de la coupe lourde produisant au moins une coupe propylène et une coupe essence dans un réacteur à écoulement ascendant avec une température de sortie réacteur comprise entre 450°C et 650°C et un rapport C/O compris entre 2 et 20, ou dans un réacteur à écoulement descendant avec une température de sortie du réacteur comprise entre 480°C et 650°C et un rapport C/O compris entre 10 et 50, et en présence d'un catalyseur constitué d'une matrice d'alumine, de silice ou de silice alumine, dans lequel :
- lorsque le procédé fonctionne en marche maxi propylène, ladite coupe essence produite par ladite étape de craquage catalytique est une coupe essence C5- et de point final entre 150°C et 220°C, le craquage catalytique fonctionnant à une température de sortie supérieure à 600°C, et le catalyseur contenant une certaine proportion de zéolithe ZSM5,
- lorsque le procédé fonctionne en marche maxi distillat, ladite coupe essence produite par ladite étape de craquage catalytique est une coupe essence C5-150°C,
b) une étape d'hydrogénation sélective (SHU) de ladite coupe essence produite par ladite étape de craquage catalytique (FCC) fonctionnant aux conditions suivantes: une pression comprise entre 0,5 et 5 MPa, une température comprise entre 80°C et 220°C, avec une vitesse spatiale liquide (LHSV) comprise entre 1h⁻¹ et 10 h⁻¹, la vitesse spatiale liquide étant exprimée en litre de charge par litre de catalyseur et par heure (l/l.h), et en présence d'un catalyseur comprenant au moins un métal du groupe VIb et au moins un métal non noble du groupe VIII déposés sur un support poreux, et dans lequel la teneur en poids d'oxyde de l'élément du groupe VIb est comprise entre 4 et 20% poids, et la teneur en poids d'oxyde de l'élément du groupe VIII est inférieure à 15% poids, l'étape d'hydrogénation sélective (SHU) permettant l'hydrogénation des dioléfines contenues dans ladite coupe essence et la conversion des composés soufrés légers de ladite coupe essence en composés soufrés de point d'ébullition supérieur,
c) une étape de séparation par distillation (SPLIT) de l'essence issue de l'étape b) permettant de séparer deux coupes: une coupe essence légère C5-Pf, et une coupe essence lourde Pf-220°C, la température Pf faisant la démarcation entre essence légère et essence lourde étant comprise entre 50°C et 80°C,
d) une étape de purification (PUR) de l'essence légère C5-Pf issue de l'étape c) qui a pour but d'abattre l'azote à moins de 1 ppm poids, de préférence moins de 0,2 ppm par contact avec un adsorbant choisi parmi les alumines, les silices alumines et les tamis moléculaires,
e) une étape d'oligomérisation (OLG) de l'essence légère C5-Pf issue de l'étape de purification (PUR), les conditions opératoires de ladite étape d'oligomérisation (OLG) étant les suivantes:
lorsque le procédé fonctionne en marche maxi propylène :
- température comprise entre 120°C et 250°C,
- pression comprise entre 3 et 6 MPa,
- catalyseurs à base de silice alumine,
lorsque le procédé fonctionne en marche maxi distillât :
- température comprise entre 150°C et 350°C,
- pression comprise entre 3 et 6 MPa,
- catalyseur à base zéolithe cristallisée,
lesdites conditions opératoires permettant d'orienter la production vers l'essence ou le distillat moyen,
f) une étape de séparation des oligomères obtenus à l'issue de l'étape e) permettant de dégager au moins 2 coupes :
- une coupe essence légère PI-150°C,
- une coupe essence lourde 150°C-220°C, et
- une coupe 220°C+,
le procédé fonctionnant en outre selon les caractéristiques suivantes :
- lorsque le procédé fonctionne en marche maxi propylène, la coupe essence légère PI-150°C, la coupe essence lourde 150°C-220°C et la coupe 220°C+ issues de l'étape de séparation des oligomères sont recyclées au FCC,
- lorsque le procédé fonctionne en marche maxi distillat, la coupe essence légère PI-150°C est recyclée au FCC.

2. Procédé de conversion d'une charge hydrocarbonée lourde présentant une grande flexibilité pour la production de distillat moyen, d'essence et de propylène selon la revendication 1, dans lequel, lorsque le procédé fonctionne en marche maxi distillat, le catalyseur de l'unité d'oligomérisation est sélectionné dans le groupe constitué par les zéolithes ferrierite, ZSM-5, Mordénite et ZSM-22, prises seules ou en mélange, et de façon très préférée la zéolithe utilisée étant la ZSM-5.

3. Procédé de conversion d'une charge hydrocarbonée lourde présentant une grande flexibilité pour la production de distillat moyen, d'essence et de propylène selon la revendication 1, dans lequel l'unité de purification (PUR) située en amont de l'unité d'oligomérisation (OLG) fait appel à un lavage à l'eau préalable, suivi d'une adsorption sur des adsorbants tels que les alumines, les silices alumines ou les tamis moléculaires, ces derniers étant préférentiellement à base de zéolithes de type NaX ou NaY.

## Patentansprüche

1. Verfahren zur Umwandlung eines schweren Kohlenwasserstoff-Einsatzstoffs, der aus Verbindungen mit Siedepunkten von mehr als 340 °C besteht, wobei das Verfahren eine große Flexibilität für die Herstellung von Propylen, Benzin und Mitteldestillat aufweist, wobei die folgenden Schritte angewendet werden, wenn das Verfahren in Maxi-Propylen- oder Maxi-Destillat-Fahrweise arbeitet:
a) ein Schritt des katalytischen Crackens (FCC) des schweren Schnitts unter Produktion mindestens eines Propylen-Schnitts und eines Benzin-Schnitts in einem Reaktor mit aufsteigender Strömung mit einer Reaktorausgangstemperatur zwischen 450 °C und 650 °C und einem C/O-Verhältnis zwischen 2 und 20 oder in einem Reaktor mit absteigender Strömung mit einer Reaktorausgangstemperatur zwischen 480 °C und 650 °C und einem C/O-Verhältnis zwischen 10 und 50 und in Gegenwart eines Katalysators, der aus einer Aluminiumoxid-, Siliciumdioxid- oder Siliciumdioxid-Aluminiumoxid-Matrix besteht, wobei:
- dann, wenn das Verfahren in Maxi-Propylen-Fahrweise arbeitet, der durch den Schritt des katalytischen Crackens produzierte Benzin-Schnitt ein C5-Benzinschnitt mit einem Endpunkt zwischen 150 °C und 220 °C ist, wobei das katalytische Cracken bei einer Ausgangstemperatur von mehr als 600 °C arbeitet und der Katalysator einen bestimmten Anteil an ZSM5-Zeolith enthält,
- dann, wenn das Verfahren in Maxi-Destillat-Fahrweise arbeitet, der durch den Schritt des katalytischen Crackens produzierte Benzin-Schnitt ein C5-150°C-Benzinschnitt ist,
b) ein Schritt der selektiven Hydrierung (SHU) des durch den Schritt des katalytischen Crackens (FCC) produzierten Benzin-Schnitts, der unter den folgenden Bedingungen arbeitet: Druck zwischen 0,5 und 5 MPa, Temperatur zwischen 80 °C und 220 °C, mit einer Katalysatorbelastung (LHSV) zwischen 1 h⁻¹ und 10 h⁻¹, wobei die Katalysatorbelastung in Liter Einsatzstoff pro Liter Katalysator und pro Stunde (l/l.h) ausgedrückt wird, und in Gegenwart eines Katalysators, der mindestens ein Metall der Gruppe VIb und mindestens ein Nichtedelmetall der Gruppe VIII, die auf einem porösen Träger abgeschieden sind, umfasst, wobei der Gewichtsgehalt an Oxid des Elements der Gruppe VIb zwischen 4 und 20 Gew.-% liegt und der Gewichtsgehalt an Oxid des Elements der Gruppe VIII weniger als 15 Gew.-% beträgt, wobei der Schritt der selektiven Hydrierung (SHU) die Hydrierung der in dem Benzin-Schnitt enthaltenen Diolefine und die Umwandlung der leichten Schwefelverbindungen des Benzin-Schnitts in Schwefelverbindungen mit höherem Siedepunkt erlaubt,
c) einen Schritt der destillativen Trennung (SPLIT) des Benzins aus Schritt b), der die Trennung von zwei Schnitten erlaubt: einem leichten C5-Pf-Benzin-Schnitt und einem schweren Pf-220°C-Benzin-Schnitt, wobei die Temperatur Pf, die Abgrenzung zwischen leichtem Benzin und schwerem Benzin bildet, zwischen 50 °C und 80 °C liegt,
d) einen Schritt der Reinigung (PUR) des leichten C5-Pf-Benzin-Schnitts aus Schritt c) zur Verringerung des Stickstoffs auf weniger als 1 Gew.-ppm, vorzugsweise weniger als 0,2 ppm, durch Kontakt mit einem aus Aluminiumoxiden, Siliciumoxid-Aluminiumoxiden und Molsieben ausgewählten Adsorptionsmittel,
e) einen Schritt der Oligomerisation (OLG) des leichten C5-Pf-Benzins aus dem Reinigungsschritt (PUR), wobei die Arbeitsbedingungen für den Oligomerisationsschritt (OLG) wie folgt sind:
dann, wenn das Verfahren in Maxi-Propylen-Fahrweise arbeitet:
- Temperatur zwischen 120 °C und 250 °C,
- Druck zwischen 3 und 6 MPa,
- Katalysatoren auf Basis von Siliciumoxid-Aluminiumoxid,
dann, wenn das Verfahren in Maxi-Destillat-Fahrweise arbeitet:
- Temperatur zwischen 150 °C und 350 °C,
- Druck zwischen 3 und 6 MPa,
- Katalysator auf Basis von kristallisiertem Zeolith,
wobei die Arbeitsbedingungen es erlauben, die Produktion in Richtung Benzin oder Mitteldestillat zu orientieren,
f) einen Schritt der Abtrennung der am Ende von Schritt e) erhaltenen Oligomere, was die Befreiung von mindestens 2 Schnitten erlaubt:
- einem leichten PI-150°C-Benzin-Schnitt,
- einem schweren 150°C-220°C-Benzin-Schnitt und
- einem 220°C+-Schnitt,
wobei das Verfahren außerdem gemäß den folgenden Merkmalen arbeitet:
- dann, wenn das Verfahren in Maxi-Propylen-Fahrweise arbeitet, werden der leichte Pl-150°C-Benzin-Schnitt, der schwere 150°C-220°C-Benzin-Schnitt und der 220°C+-Schnitt aus dem Schritt der Abtrennung der Oligomere zum FCC zurückgeführt,
- dann, wenn das Verfahren in Maxi-Destillat-Fahrweise arbeitet, wird der leichte Pl-150°C-Benzin-Schnitt zum FCC zurückgeführt.

2. Verfahren zur Umwandlung eines schweren Kohlenwasserstoff-Einsatzstoffs mit großer Flexibilität für die Herstellung von Mitteldestillat, Benzin und Propylen nach Anspruch 1, wobei dann, wenn das Verfahren in Maxi-Destillat-Fahrweise arbeitet, der Katalysator der Oligomerisationeinheit aus der Gruppe bestehend aus Ferrierit-, ZSM-5-, Mordenit- und ZSM-22-Zeolithen, alleine oder in einer Mischung, ausgewählt wird und besonders bevorzugt ZSM-5 als Zeolith verwendet wird.

3. Verfahren zur Umwandlung eines schweren Kohlenwasserstoff-Einsatzstoffs mit großer Flexibilität für die Herstellung von Mitteldestillat, Benzin und Propylen nach Anspruch 1, wobei in der Reinigungseinheit (PUR), die sich stromaufwärts der Oligomerisationseinheit (OLG) befindet, eine Vorwäsche mit Wasser gefolgt von einer Adsorption an Adsorptionsmitteln wie Aluminiumoxiden, Siliciumdioxid-Aluminiumoxiden oder Molsieben angewendet wird, wobei Letztere vorzugsweise auf Zeolithen vom NaX- oder NaY-Typ basieren.

## Claims

1. Process for converting a heavy hydrocarbon feed consisting of compounds having boiling points of more than 340°C, said process presenting a high flexibility for the production of propylene, gasoline and middle distillate, employing the following steps when the process is operating in maxi propylene or maxi distillate mode:
a) a step of catalytic cracking (FCC) of the heavy cut, producing at least a propylene cut and a gasoline cut in an ascending-flow reactor with a reactor exit temperature of between 450°C and 650°C and a C/O ratio of between 2 and 20, or in a descending-flow reactor with a reactor exit temperature of between 480°C and 650°C and a C/O ratio of between 10 and 50, and in the presence of a catalyst consisting of an alumina, silica or silica-alumina matrix, wherein:
- when the process is operating in maxi propylene mode, said gasoline cut produced by said catalytic cracking step is a C5- gasoline cut with a final point between 150°C and 220°C, the catalytic cracking operating at an exit temperature of more than 600°C, and the catalyst containing a certain proportion of zeolite ZSM5,
- when the process is operating in maxi distillate mode, said gasoline cut produced by said catalytic cracking step is a C5-150°C gasoline cut,
b) a step of selective hydrogenation (SHU) of said gasoline cut produced by said catalytic cracking step (FCC), operating under the following conditions: a pressure of between 0.5 and 5 MPa, a temperature of between 80°C and 220°C, with a liquid hourly space velocity (LHSV) of between 1 h⁻¹ and 10 h⁻¹, the liquid hourly space velocity being expressed as litres of charge per litre of catalyst per hour (l/l.h), and in the presence of a catalyst comprising at least one group Vlb metal and at least one non-noble group VIII metal, applied to a porous support, and wherein the weight content of oxide of the group Vlb element is between 4 and 20 weight%, and the weight content of oxide of the group VIII element is less than 15 weight%, the selective hydrogenation step (SHU) allowing the hydrogenation of the diolefins present in said gasoline cut and the conversion of the light sulfur compounds from said gasoline cut into sulfur compounds of higher boiling point,
c) a step of distillative separation (SPLIT) of the gasoline obtained from step b), allowing separation of two cuts: a light C5-Pf gasoline cut, and heavy Pf-220°C gasoline cut, the temperature Pf forming the boundary between light gasoline and heavy gasoline being between 50°C and 80°C,
d) a step of purifying (PUR) the light C5-Pf gasoline cut obtained from step c), with the aim of diminishing the nitrogen to less than 1 ppm by weight, preferably less than 0.2 ppm, by contact with an adsorbent selected from aluminas, silica-aluminas and molecular sieves,
e) a step of oligomerizing (OLG) the light C5-Pf gasoline cut obtained from the purification step (PUR), the operating conditions of said oligomerization step (OLG) being as follows:
when the process is operating in maxi propylene mode:
- temperature between 120°C and 250°C,
- pressure between 3 and 6 MPa,
- catalysts based on silica-alumina;
when the process is operating in maxi distillate mode:
- temperature between 150°C and 350°C,
- pressure between 3 and 6 MPa,
- catalyst based on crystalline zeolite,
said operating conditions allowing production to be oriented towards gasoline or middle distillate,
f) a step of separating the oligomers obtained at the end of step e), allowing the freeing of at least two cuts:
- a light PI-150°C gasoline cut,
- a heavy 150°C-220°C gasoline cut, and
- a 220°C+ cut,
the process operating further according to the following features:
- when the process is operating in maxi propylene mode, the light PI-150°C gasoline cut, the heavy 150°C-220°C gasoline cut and the 220°C+ cut obtained from the oligomer separation step are recycled to the FCC,
- when the process is operating in maxi distillate mode, the light PI-150°C gasoline cut is recycled to the FCC.

2. Process for converting a heavy hydrocarbon feed, presenting a high flexibility for the production of middle distillate, gasoline and propylene, according to Claim 1, wherein, when the process is operating in maxi distillate mode, the catalyst of the oligomerization unit is selected from the group consisting of the zeolites ferrierite, ZSM-5, mordenite and ZSM-22, alone or in a mixture, the zeolite used being very preferably ZSM-5.

3. Process for converting a heavy hydrocarbon feed, presenting a high flexibility for the production of middle distillate, gasoline and propylene, according to Claim 1, wherein the purification unit (PUR) sited upstream of the oligomerization unit (OLG) employs a preliminary water wash, followed by an adsorption on adsorbents such as aluminas, silica-aluminas or molecular sieves, the latter being preferably based on NaX or NaY zeolites.
